# DEMANDE DE BREVET EUROPEEN

(11) **EP 0 524 874 A1**
(43) Date de publication de la demande: **27.01.1993**
(21) Numéro de dépôt: 92402102.5
(22) Date de dépôt: 21.07.1992
(51) Int. Cl.: A61F 2/30, A61F 2/40, A61F 2/42

(54) **Prothèse articulaire, notamment trapézo-métacarpienne et digitale**

(30) Priorité: 24.07.1991 FR 9109377
(71) Demandeur: HADES, F-80000 Amiens (FR)
(72) Inventeur: Obry, Christophe, F-80680 Saint Fuscien (FR); Sueur, Patrick, F-80000 Amiens (FR); Letot, Patrick, F-76200 Dieppe (FR); Hiot, Jean-Claude, F-80300 Bouzincourt (FR)
(74) Mandataire: Martin, Jean-Paul

(57) **Abrégé**

Cette prothèse (8) comprend :
- deux parties en matériau biocompatible (9, 11) destinées à être ancrées chacune dans l'un (6, 7) des os de l'articulation,
- et une troisième partie formée par un piston (12) réalisé en une matière élastique biocompatible, dont l'une des extrémités est fixée à l'une des surfaces articulaires des parties métalliques et comportant une tige (18) qui peut librement coulisser dans une cavité (11a) ménagée dans l'autre surface articulaire. La prothèse trapézo-métacarpienne (8) comporte une cuvette trapézienne à laquelle est fixée la tête (14) du piston (12), dont la tige (18) coulisse dans la pièce tubulaire métacarpienne (11), cette tige étant déformable dans toutes les directions. Cette prothèse présente une très grande mobilité dans toutes les directions grâce au piston déformable (12).

## Description

La présente invention a pour objet une prothèse articulaire, destinée à remplacer diverses articulations et notamment des articulations des doigts de la main.

On sait que l'arthrose affecte souvent les articulations interphalangiennes des doigts et l'articulation carpo-métacarpienne du pouce. Des modifications dégénératives produisent des proliférations osseuses autour des bords de l'articulation, et constituent souvent une localisation d'une arthrose primaire généralisée. L'articulation s'élargit et devient sensible, et la douleur peut être liée à un surmenage fonctionnel de l'articulation. Une autre indication se trouve dans les destructions de l'articulation dans les rhumatismes inflammatoires tel que la polyarthrite rhumatoïde.

Divers types de traitements plus ou moins satisfaisants sont connus, dont l'exposé n'entre pas dans le cadre du présent mémoire descriptif.On indiquera seulement que la chirurgie est indiquée si la douleur et l'incapacité sont sévères. Le choix de l'opération est orienté soit vers une arthrodèse de l'articulation, soit vers une ablation du trapèze, soit enfin vers une prothèse articulaire.

Comme on le sait, une prothèse articulaire doit satisfaire à plusieurs impératifs : restituer l'anatomie donc la fonction de l'articulation détruite, présenter une durée de vie suffisante et être biocompatible.

On a ainsi proposé un certain nombre de réalisations pour des prothèses trapézo-métacarpiennes, le plus souvent basées sur le principe de la rotule. La plupart de ces prothèses trapézo-métacarpiennes sont de conception sphéro-sphérique et souvent constituées en deux pièces, dont l'une est en métal et l'autre en plastique (en particulier le polyéthylène). Ces prothèses comportent une pièce proximale trapézienne généralement en plastique et une pièce distale métacarpienne constituée d'une queue métallique enfoncée dans le premier métacarpien. Ces deux pièces sont reliées à une rotule dont la tête est articulée dans la pièce trapézienne, celle-ci pouvant être fixée au trapèze soit par ostéo-intégration grâce à une forme appropriée, soit au moyen d'un ciment biologique.

Ces prothèses connues ne donnent pas entière satisfaction, car elles finissent par se désceller ou par casser après une certaine durée de service, ou encore subissent des luxations, en raison des contraintes en flexion, en translation et en torsion qu'elles subissent.

De plus, les pièces en plastique utilisées, telles que des élastomères de silicone, provoquent une réaction du tissu avec lequel elles sont en contact et des troubles corrélatifs gênants pour la personne portant cette prothèse.

Enfin, en plus des inconvénients ci-dessus les prothèses connues ont une liberté de débattement limitée à celle d'une articulation à rotule, donc très inférieure aux multiples degrés de liberté d'une articulation naturelle.

L'invention a donc pour but de proposer une prothèse articulaire ne présentant pas les inconvénients ci-dessus des prothèses connues.

Conformément à l'invention, la prothèse articulaire comprend : une première partie en matériau biocompatible destinée à être ancrée dans l'un des os de l'articulation, une seconde partie en matériau biocompatible destinée à être ancrée dans l'autre os de l'articulation, ces deux parties pouvant présenter chacune une surface articulaire, et une troisième partie formée par un piston réalisé en une matière élastique biocompatible, reliant les surfaces articulaires, dont l'une des extrémités peut être fixée à l'une des surfaces articulaires et qui peut librement coulisser dans une cavité ménagée dans l'autre surface articulaire.

L'invention sera décrite ci-après en référence aux dessins annexés qui en illustrent plusieurs formes de réalisation à titre d'exemples non limitatifs.

La figure 1 est une vue en perspective du squelette d'une main humaine montrant ses diverses articulations et notamment l'articulation carpo-métacarpienne du pouce, assurée par une prothèse selon une première forme de réalisation de l'invention.

La figure 2 est une vue en coupe axiale à échelle agrandie d'une prothèse articulaire trapézo-métacarpienne selon cette première forme de réalisation de l'invention.

Les figures 3 et 4 sont des vues en coupe axiale de deux variantes de réalisation de la prothèse de la Fig.2.

La figure 5 est une vue en perspective d'une prothèse articulaire trapézo-métacarpienne selon une seconde forme de réalisation de l'invention.

La figure 6 est une vue en élévation à échelle agrandie, d'une prothèse articulaire digitale mise en place dans une première phalange et un métacarpien, selon une troisième forme de réalisation. Celle-ci est destinée aux articulations métacarpophalangiennes des 5 doigts et aux interphalangiennes proximales des 4 doigts longs.

La figure 7 est une section suivant 7/7 de la Fig.6.

La figure 8 est une vue en perspective d'une prothèse articulaire temporo-mandibulaire suivant une quatrième forme de réalisation de l'invention.

La figure 9 est une vue en perspective éclatée des différentes pièces constitutives de la prothèse de la Fig.6.

La figure 10 est une vue en coupe axiale d'une prothèse articulaire astragalo-calcanéenne suivant une cinquième forme de réalisation de l'invention, placée dans l'articulation correspondante.

La figure 11 est une vue en perspective à échelle agrandie par rapport à la Fig.8 du piston en matière plastique de la prothèse.

La figure 12 est une vue en perspective avec coupe axiale partielle d'une prothèse articulaire scapulo-humérale selon une sixième forme de réalisation de l'invention.

La figure 13 est une vue en perspective partielle de la prothèse de la Fig.12.

La figure 14 est une vue en coupe partielle de la pièce scapulaire de la prothèse de la Fig.12 et en élévation de la tige du piston.

La figure 15 est une vue de dessus avec coupe partielle, à échelle réduite, d'une prothèse articulaire radio-carpienne selon une septième forme de réalisation de l'invention.

La figure 16 est une vue en élévation des deux parties constitutives de la pièce carpienne de la prothèse de la Fig.11.

Les figures 17 et 18 sont des vues en élévation suivant les directions B et A des deux éléments de la pièce carpienne représentée à la Fig.16.

La figure 19 est une vue en coupe longitudinale de la partie radiale de la prothèse de la Fig.15.

La figure 20 est une vue de dessous de la pièce radiale de la Fig.19.

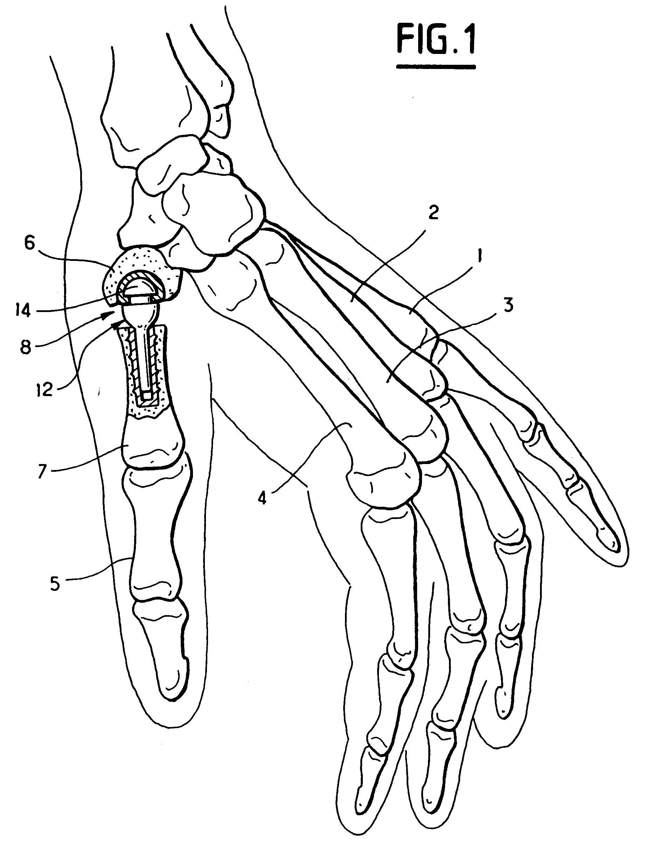

On voit à la Fig.1 les articulations des doigts 1, 2, 3, 4 et du pouce 5 d'une main humaine, ainsi qu'une prothèse trapézo-métacarpienne 8 assurant l'articulation entre le trapèze 6 et le premier métacarpien 7 du pouce.

Cette prothèse 8 est réalisée pour remplacer l'articulation trapézo-métacarpienne du pouce détruite. Elle comprend une première partie en matériau biocompatible 9 en forme de cupule ou de cuvette, hémisphérique et adaptée pour être ancrée dans le trapèze 6, une seconde partie 11 également en matériau biocompatible, destinée à être ancrée dans le premier métacarpien 7, constituée d'une pièce tubulaire, de préférence conique, et enfin une troisième partie formée par un piston 12 réalisé en une matière élastique biocompatible, telle qu'un élastomère de silicone.

La pièce tubulaire 11 peut être fixée à l'intérieur du premier métacarpien 7 par tous moyens appropriés, par exemple des picots superficiels 13. Le piston 12 comporte une tête 14 qui remplit la cuvette 9 et peut être maintenue fixée dans celle-ci par une collerette 15 saillant radialement sur le pourtour de la cuvette 14 et qui s'engage dans une gorge complémentaire 16 formée dans la tête 14. Au-delà de la gorge 16, le piston 12 peut se prolonger par un corps 17 et une tige 18 qui s'étend à l'intérieur de la pièce distale 11; la pénétration de la tige 18 dans la pièce 11 est dans ce cas limitée par un épaulement 19 du corps 17, pouvant venir en appui sur l'extrémité de la pièce 11.

La tige 18 peut librement coulisser dans le canal interne 11a de la pièce 11, et peut se déformer par pliage durant les déplacements relatifs de la prothèse proximale 9 et de la prothèse distale 11.

Grâce à la présence des composants 9 et 11, le piston élastique 12 est préservé de tout contact avec l'os, ce qui prévient d'éventuels risques de réactions tissulaires telle que la "siliconite". Cette prothèse présente de multiples degrés de liberté dans toutes les directions, le degré de liberté longitudinal par translation dans la pièce conique 11 n'étant limité que par les ligaments (non représentés) de l'articulation. La cupule trapézienne 9 peut être fixée au trapèze 6 avec ou sans ciment biologique, ce ciment étant superflu si la forme de la cuvette 9 permet son ostéo-intégration. La pièce conique est ajustée au niveau de la base du premier métacarpien 7, plutôt que scellée, ce qui autorise plus facilement les reprises, ou d'éventuels arthodèses secondaires. Le composant distal 11 est en contact avec le premier métacarpien 7 au niveau de la corticale, ce qui permet d'éviter les déplacements de ce composant 11 en varus.

La prothèse 8 autorise l'ensemble des mouvements de l'articulation, et non plus seulement les mouvements du type rotule comme les prothèses connues jusqu'à présent, et ce dans les amplitudes habituelles de l'articulation trapézo-métacarpienne : rotations, abductions, adductions, ante-pulsions, rétro-pulsions. Cette prothèse s'adapte aux destructions articulaires provenant des causes suivantes : rhizarthrose, arthrite rhumatoïde, dislocation post-traumatique.

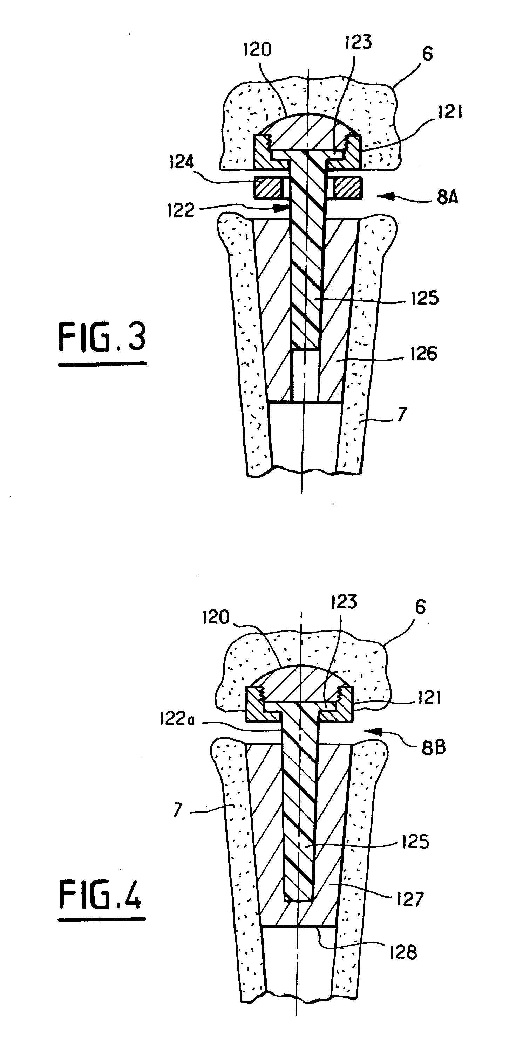

La forme d'exécution de la prothèse trapézo-métacarpienne 8A représentée à la Fig.3 comporte une coiffe trapézienne 120 vissée dans une embase 121 et qui comprime la tête 123 du piston élastique 122, l'empêchant ainsi de sortir de la coiffe 120. Une pastille 124 traversée axialement par la tige 125 du piston 122 est interposée entre l'embase 121 et la partie tubulaire métacarpienne 126, et limite la pénétration de la tige 125 dans cette dernière.

Dans la prothèse 8B de la Fig.4, la pastille 124 a été supprimée, et la limitation de la pénétration du piston 122 dans le canal de la pièce distale borgne 127 est assurée par le fond 128 de celle-ci. La venue en butée de la tige 125 contre le fond 128 provoque alors une compression et un gonflement de la partie 122a du piston 122 située entre les pièces 121 et 127.

Les prothèses 8A et 8B présentent les mêmes avantages que la prothèse 8.

En plus des avantages déjà mentionnés, la prothèse selon l'invention offre moins de risques de déplacements secondaires, en particulier en varisation du composant métacarpien qui provoque un balayage de la partie distale dans le canal médullaire.. En outre il existe moins de contraintes au niveau des pièces scellées 9 ou impactées 11, grâce à la présence du piston déformable 12 et à l'amortissement des contraintes autorisées par celui-ci. Ainsi est limité le risque de descellement de la prothèse 8.

Cette dernière est également moins encombrante que les précédentes, car l'articulation s'effectue par l'intermédiaire d'un piston déformable, et non par une pièce rigide, non déformable. Enfin cette prothèse trapézo-métacarpienne limite l'importance des coupes osseuses nécessaires grâce à son encombrement réduit, et respecte ainsi le capital osseux. Cet avantage est important en cas de reprise chirurgicale par une nouvelle arthroplastie ou une arthrodèse trapézo-métacarpienne.

La mise en place de la prothèse trapézo-métacarpienne 8 qui vient d'être décrite s'effectue par le chirurgien de la manière suivante :
La voie d'abord postéro-externe est conseillée, avec un bord entre le long extenseur du pouce et le long abducteur du pouce. Il convient de respecter soigneusement les rameaux sensitifs du nerf radial, et de repérer les vaisseaux radiaux.

Une dissection capsulaire est exécutée juste au-delà des surfaces articulaires, avec respect si possible du tendon du long abducteur du pouce, au niveau de son insertion (l'économie du capital osseux étant importante à considérer).

Le chirurgien creuse le premier métacarpien 7 et repère la cavité centro-médullaire avec une pointe carrée et une petite curette. Il creuse la base du premier métacarpien à l'aide de la curette, approximativement selon la taille de la prothèse. L'ajustement du creusement est terminé avec l'ancillaire de mise en place (de la taille appropriée au patient). Le calque pré-opératoire aura préalablement permis de déterminer la prothèse à utiliser.

Le chirurgien se porte ensuite au niveau du trapèze où il réalise à la curette plutôt qu'à la fraise, un creusement d'un diamètre d'approximativement 10mm. La curette est préférée à la fraise, qui risque d'entraîner des nécroses osseuses. Le moteur doit être utilisé seulement en complément pour ajuster le creusement. Il est utile de laisser et de maintenir la corticale externe du trapèze, pour ne pas affaiblir cette partie du trapèze qui supportera le plus de contraintes de la pince pollici-digitale. Le chirurgien met alors en place la prothèse d'essai. L'orientation de la cupule est capitale.

Il procède ensuite à l'impaction, à l'aide de l'ancillaire prévu à cet effet, de la pièce métacarpienne métallique 11 qui peut dépasser de 0,5mm de la base du premier métacarpien 7.

La pièce trapézienne 9 est alors scellée avec du ciment biologique. L'ajustement de la mise en place s'effectue à l'aide de la pince prévue à cet effet. Il convient de vérifier l'orientation de la pièce trapézienne 9, qui doit être parallèle au plan de la prothèse métacarpienne 11 et à l'axe du scaphoïde. Ce cotyle en métal peut également déborder le plan du trapèze 9 de 0,5mm. La tête 14 du piston 12 est introduite dans la cupule 9, grâce à des pinces mousses afin de ne pas traumatiser le piston 12. Il est conseillé d'effectuer un geste de renforcement capsulaire externe, afin de lutter contre la subluxation externe, et pour soulager la prothèse des contraintes en cisaillement, qui s'effectue au niveau du piston 12.

A l'aide d'un fil bi-aiguillé, ou d'un ligament artificiel d'une force d'environ 10 déca Newtons, on réalise un point transosseux au niveau de la base du premier métacarpien versant externe. Sur ce point transosseux, le chirurgien vient amarrer solidement les éléments capsulaires externes. Il est possible de réaliser ce canal transosseux avant la mise en place de l'implant métallique métacarpien, la prothèse étant alors impactée sur le néo-ligament synthétique. Le chirurgien effectue un lâcher de garrot pour vérifier les hémostases. Enfin le chirurgien confectionne une orthèse de protection du pouce maintenant celui-ci en position de fonction, antépulsion et abduction de 40°. Cette orthèse peut être réalisée en plâtre ou en résine ou en matériau thermo-formable.

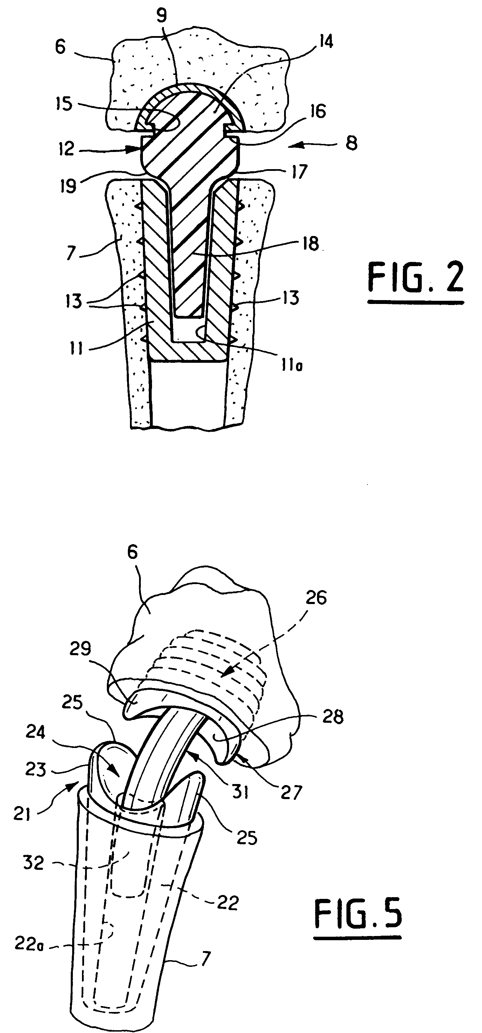

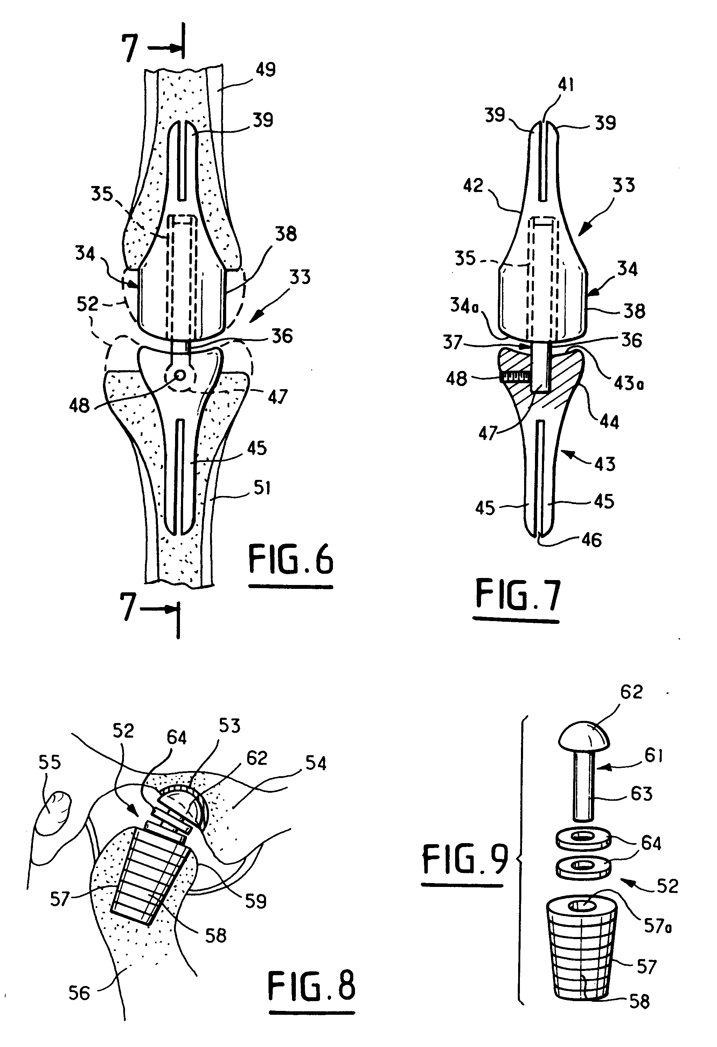

Dans la forme de réalisation de la Fig.5 de la prothèse trapézo-métacarpienne 21, celle-ci comporte une partie métacarpienne 22 dont l'extrémité proximale 23 fait saillie du premier métacarpien 7 et présente une surface sellaire 24 (en forme de selle de cheval) avec deux extrémités relevées 25. La prothèse 21 comprend également une cupule trapézienne 26 pourvue d'une extrémité distale 27 présentant une surface sellaire 28, dont les deux extrémités relevées 29 sont disposées en regard de la surface sellaire 24 suivant une direction à peu près perpendiculaire à une ligne reliant les extrémités 25, à la manière d'une articulation de cardan. Enfin le piston 31 comporte une tige 32 engagée de manière librement coulissante dans le canal interne 22a de la pièce métacarpienne 22, et dont la tête (non visible), est fixée à l'intérieur de la cuvette 26, par exemple de la même manière qu'illustrée à la Fig.2. Comme dans la réalisation précédente, le canal interne 22a a une longueur supérieure à celle de la tige 32, de manière à permettre un débattement longitudinal suffisant du piston 31.

La prothèse 33 représentée aux Fig.6 et 7 est une prothèse articulaire digitale, pouvant être utilisée pour remplacer des articulations digitales métacarpophalangiennes des cinq doigts et interphalangiennes proximales des quatre doigts longs. Elle peut également être mise en oeuvre aux articulations méta-tarso-phalangiennes des gros orteils.

La prothèse 33 comprend une pièce proximale 34 dans laquelle est ménagé un évidement allongé 35 adapté pour recevoir la tige 36 d'un piston souple 37. La pièce 34 comporte une tête distale 38 et une extrémité proximale constituée par une ou plusieurs pattes parallèles 39, au nombre de quatre dans l'exemple décrit, obtenues par une entaille longitudinale en croix 41. Les pattes 39 sont raccordées à la tête 38 par une partie intermédiaire 42 évasée.

La prothèse 33 est complétée par une pièce phalangienne 43 constituée d'une tête 44 et d'une partie distale sous la forme de doigts allongés et parallèles 45, au nombre par exemple de quatre et obtenus par une fente longitudinale 46 en croix. La tête 47 du piston 37 est ancrée dans la pièce distale 43 au moyen par exemple d'une vis latérale 48. Les deux parties proximale 34 et distale 43 présentent des surfaces de frottement respectives 34a, 43a adaptées pour coopérer ensemble lorsque la pièce proximale 34 est ajustée à l'intérieur du métacarpien 49 et la pièce distale 43 logée dans la première phalange 51. Sur la Fig.4, les traits discontinus 52 représentent les emplacements occupés par les surfaces ostéo-articulaires de l'articulation détruite, partiellement remplacées par les surfaces de frottement 34a et 43a.

Dans ce mode de réalisation, le piston déformable 37 est constitué par une simple tige.

La prothèse 52 illustrée aux Fig.8 et 9 est adaptée pour remplacer une articulation temporo-mandibulaire, la Fig.8 représentant une coupe verticale et antéro-postérieure de l'articulation temporo-maxillaire, après implantation de la prothèse 52. Cette dernière comprend une cuvette 53 sensiblement hémisphérique, scellée au moyen d'un ciment biologique dans la cavité glénoïde de l'articulation temporo-mandibulaire 54, au voisinage du conduit auditif externe 55 et au-dessus d'une branche montante 56 du maxillaire. La seconde partie 57 de la prothèse 52 est une pièce tubulaire creusée d'une cavité sensiblement cylindrique 58. La pièce 57, de préférence conique, est pourvue de filets extérieurs 58 destinés à permettre de la visser ou de l'impacter dans le condyle 59 et la branche montante 56 du maxillaire. Le piston 61 comporte une tête hémisphérique 62 venant se loger dans la cuvette 53 et fixé dans celle-ci par tous moyens appropriés, et une tige déformable 63 pouvant librement coulisser dans le canal axial 57a de la pièce 57. La prothèse 52 peut être complétée ou non par une ou plusieurs rondelles 64, par exemple au nombre de deux comme représenté, réalisées éventuellement dans le même matériau élastique que le piston 61 et qui viennent s'enfiler sur sa tige 63.

Le fonctionnement de cette prothèse est similaire à celui des prothèses précédemment décrites, les rondelles déformables 64 pouvant servir d'amortisseur entre les surfaces articulaires en regard de la cuvette 53 et de la pièce 57.

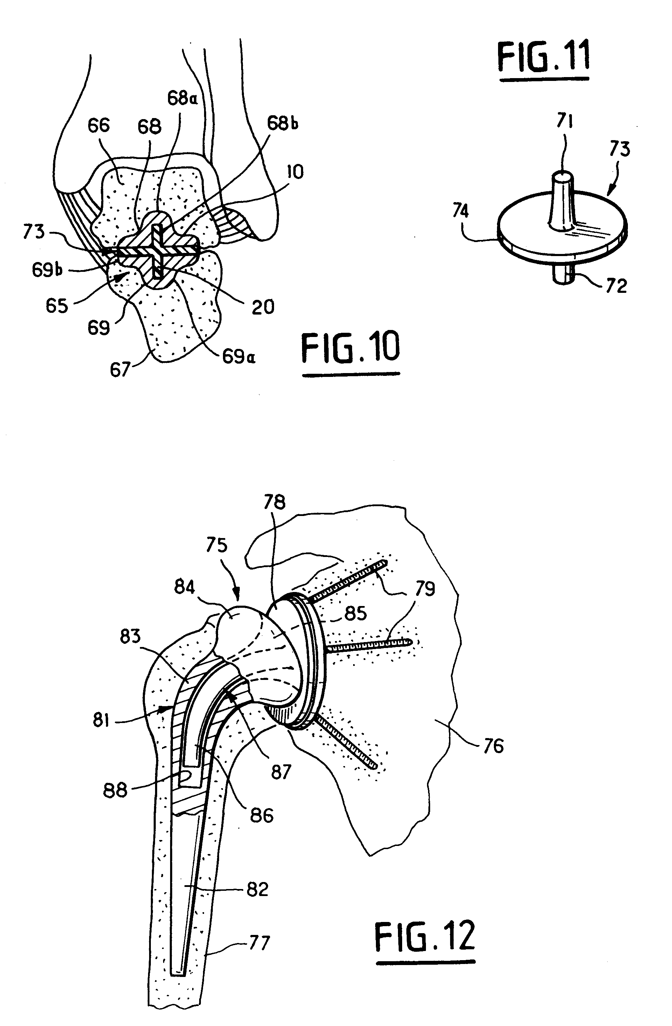

Les Fig.10 et 11 illustrent une prothèse articulaire astragalo-calcanéenne 65, destinée à remplacer une articulation détruite entre l'astragale 66 et le calcanéum 67. La prothèse 65 comprend deux parties en matériau biocompatible 68, 69 constituées chacune d'un bossage central 68a, 69a respectif et d'une base élargie 68b, 69b qui présentent chacune une surface de frottement plane, à contour circulaire, en vis-à-vis l'une de l'autre. Dans chaque bossage 68a, 69a est ménagé un puits central 10, 20 adapté pour recevoir les deux tiges opposées 71, 72 respectives d'un piston déformable 73. Ces tiges 71, 72 font saillie axialement de part et d'autre d'un disque 74 à la manière d'une toupie, et peuvent coulisser dans leur puits correspondant.

Les deux faces opposées du disque 74 coopèrent avec les surfaces articulaires planes en regard des parties 68 et 69.

La pièce 68 constitue la partie astragalienne de la prothèse, venant s'insérer dans l'astragale 66, tandis que la pièce 69 vient s'insérer dans le calcanéum 67 en vis-à-vis de la pièce 68. Comme dans les réalisations précédentes, la fixation aux os 66, 67 peut être assurée soit par ostéo-intégration soit au moyen d'un ciment biologique.

Les Fig.12 à 14 représentent une autre forme de réalisation de l'invention dans laquelle la prothèse 75 est destinée à remplacer une articulation scapulo-humérale, entre une omoplate 76 et un humérus 77. La prothèse 75 comprend une cupule glénoïdienne 78 en matériau biocompatible accompagnée ou non d'une embase 78a, pourvue de moyens de fixation à l'omoplate 76 tels que par exemple des vis 79, ou un ciment biologique, ou des plots et une pièce tubulaire 81. Cette dernière comporte une pointe 82 adaptée pour venir s'ancrer dans le canal médullaire au contact de la corticale de l'humérus 77 et, du côté de la cupule 78, une partie incurvée 83 terminée par une tête condylienne 84 évasée, présentant une ouverture centrale 85 de passage de la tige 86 d'un piston déformable 87. La cupule 78 est perçée d'une ouverture centrale de passage de la tige 86 et de retenue de la tête 90 du piston 87, encastrée dans l'embase 78a. La cupule 78 et l'embase 78a peuvent être réalisées monobloc ou en deux parties distinctes. La tige 86 est incurvée pour s'adapter à la courbure de la partie 83, et peut coulisser librement dans le canal interne 88 ménagé dans la partie incurvée 83, ce canal 88 ayant une longueur nettement supérieure à celle de la tige 86 pour permettre son débattement longitudinal.

L'ouverture centrale 85 est évasée sur son pourtour afin de permettre une mobilité suffisante du piston 87 tout en assurant une bonne congruence.

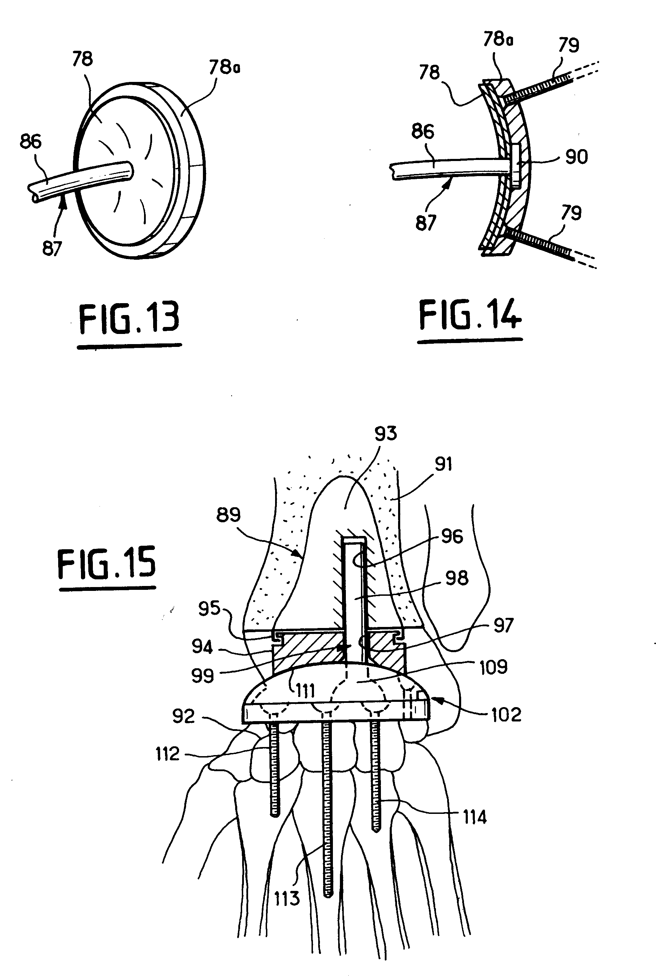

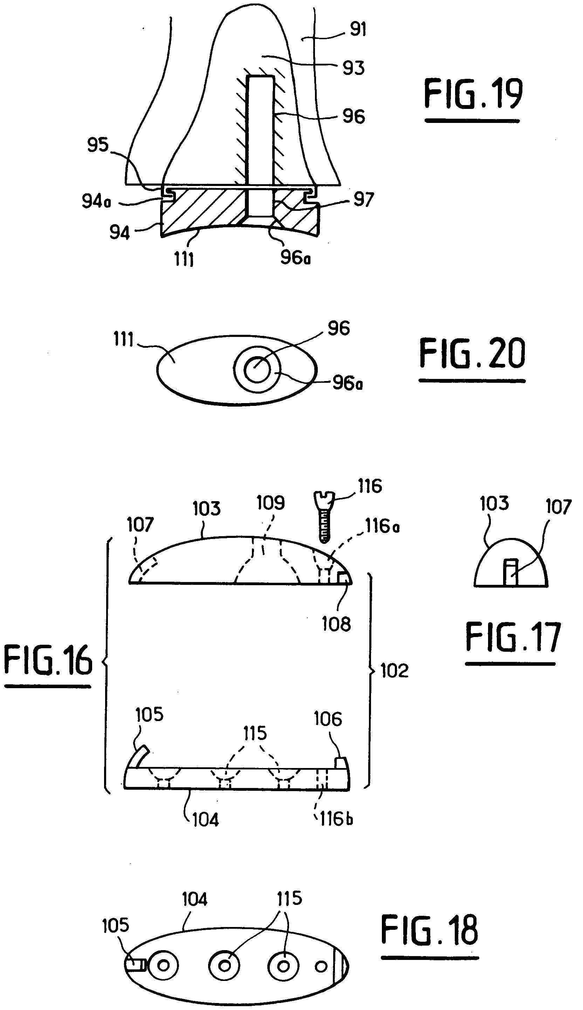

Les Fig.15 à 20 représentent une prothèse articulaire radio-carpienne 89, destinée à remplacer une articulation entre le radius 91 et le carpe 92. Cette prothèse 89 comporte une embase radiale 93 en matériau biocompatible et un plateau glénoïdien 94 fixé à la pièce 93 par tout moyen approprié, par exemple par des crochets 95 de la pièce 93 venant s'engager dans des dégagements correspondants 94a prévus sur le plateau 94. Deux puits 96, 97 sont ménagés respectivement dans les pièces 93 et 94 en regard l'un de l'autre, afin de recevoir la tige 98 d'un piston élastique déformable 99 pourvu d'une tête élargie 101. Le plateau glénoïdien 94 dispose d'une ouverture évasée sur la partie distale du puits 96 pour permettre un débattement transversal de la tige 98.

La seconde partie de la prothèse 89 est une pièce carpienne 102 constituée par l'assemblage d'un secteur sphérique 103 et d'une embase 104 par tout moyen approprié, par exemple des crochets ou ergots 105, 106 saillant de l'embase 104 et venant s'engager dans des logements complémentaires 107, 108 du secteur 103. L'assemblage des pièces 103, 104 est complété par une vis 116 pouvant être vissée dans des trous taraudés 116a et 116b desdites pièces. Dans le secteur 103 est ménagé un trou central 109 de réception de la tête 101 du piston 99.

Le plateau glénoïdien 84 et le secteur 103 présentent des surfaces de frottement mutuel complémentaires, à savoir une surface concave 111 pour le plateau 94 et la surface convexe sphérique du secteur 103.

Le plateau glénoïdien 94 est également avantageusement réalisé en un matériau favorisant les conditions de frottement avec la surface sphérique du secteur 103. La pièce carpienne 102 est fixée au carpe par des vis 112, 113, 114 dont les têtes sont ancrées dans des évidements 115 de l'embase 104.

Dans les diverses formes de réalisation de la prothèse articulaire selon l'invention, celles-ci présentent avantageusement une mobilité complexe dans toutes les directions grâce aux pistons déformables, biocompatibles, en évitant d'une'part la luxation, première cause de complications dans les prothèses dites non contraintes, et d'autre part les sollicitations excessives dans les ancrages osseux, provoquant les descellements, première cause de complications dans les prothèses dites contraintes.

Par ailleurs, les surfaces en frottement mutuel du piston déformable et de la partie en matériau biocompatible avec laquelle ce piston frotte, peuvent être avantageusement recouvertes d'un matériau à faible coefficient de frottement, par exemple du carbone pyrolytique. Dans la réalisation de la Fig.2 il en sera ainsi de la tige 18 et de la surface du canal interne 11a; dans la réalisation de la Fig.3 seront concernées la tige 32 et la surface du canal interne 22a etc...

## Revendications

1. Prothèse articulaire (8, 21...), caractérisée en ce qu'elle comprend en combinaison :
- une première partie en matériau biocompatible (9; 34...) destinée à être ancrée dans l'un des os de l'articulation,
- une seconde partie en matériau biocompatible (11; 22; 44...) destinée à être ancrée dans l'autre os de l'articulation,
- ces deux parties pouvant présenter chacune une surface articulaire,
- et une troisième partie formée par un piston (12; 31; 37...) réalisé en une matière élastique biocompatible, reliant les surfaces articulaires, dont l'une des extrémités peut être fixée à l'une des surfaces articulaires et qui peut librement coulisser dans une cavité (11a, 22a,...) ménagée dans l'autre surface articulaire.

2. Prothèse (8) selon la revendication 1, destinée à remplacer une articulation trapézo-métacarpienne, caractérisée en ce que la première partie est une cuvette en matériau biocompatible (9) adaptée pour être ancrée dans le trapèze (6), la seconde partie est une pièce tubulaire (11) en matériau biocompatible, de préférence conique, destinée à être introduite dans le premier métacarpien (7), et le piston (12) comporte une tête (14) ancrée dans la cuvette et une tige (18) s'étendant librement dans le canal interne (11a) de la pièce tubulaire, cette tige étant déformable dans toutes les directions et ayant un degré de liberté longitudinale (Fig.2).

3. Prothèse selon la revendication 2, caractérisée en ce qu'elle comporte des moyens de retenue de la cuvette (9) sur la tête (14) du piston (12), par exemple une collerette (15) saillante radialement sur le pourtour de la cuvette et qui s'engage dans une gorge complémentaire (16) formée dans la tête du piston.

4. Prothèse (21) selon la revendication 3, caractérisée en ce que les extrémités en vis-à-vis de la cuvette trapézienne (26) et de la pièce tubulaire métacarpienne (22) sont réalisées de manière à présenter des surfaces complémentaires (25, 28) en forme de selle, orientées suivant des directions sensiblement perpendiculaires, améliorant le frottement de ces deux pièces l'une sur l'autre (Fig.5).

5. Prothèse selon l'une quelconque des revendications 2 à 4, caractérisée en ce qu'elle comprend un ciment biologique de scellement de la cuvette (9, 26...) dans le trapèze (6), la fixation de la pièce tubulaire (11, 22) dans le premier métacarpien (7) étant assurée par exemple par des picots (13) ou par ajustement de cette pièce à la base du premier métacarpien.

6. Prothèse (33) selon la revendication 1, destinée aux articulations digitales, à savoir aux métacarpophalangiennes des cinq doigts, aux interphalangiennes proximales et distale de tous les doigts, aux méta-tarso-phalangiennes des gros orteils, caractérisée en ce que chacune desdites première et seconde parties (34, 43) est formée d'une pièce évidée et évasée, adaptée pour recevoir une partie d'une tige déformable (36) du piston (37), et cette pièce comporte une ou plusieurs pattes (39, 45) destinées à venir s'ancrer dans les os (49, 51) respectifs de l'articulation (Fig.6-7).

7. Prothèse (52) selon la revendication 1, destinée à une articulation temporo-mandibulaire, caractérisée en ce que la première partie est une cuvette (53) sensiblement hémisphérique, destinée à être placée dans une cavité glénoïde temporale (53), la seconde partie est une pièce tubulaire (57) creusée d'une cavité (58) sensiblement cylindrique destinée à être vissée ou impactée dans un condyle (59) de la branche montante (56) d'un maxillaire, et le piston (61) comprend une tige déformable (63) pouvant librement coulisser dans la cavité cylindrique (58) et dont une tête (62) est fixée dans la cuvette (Fig.8-9).

8. Prothèse selon la revendication 7, caractérisée en ce que le piston (61) est pourvu ou non d'au moins une rondelle (64) en matériau élastique biocompatible, montée librement coulissante sur la tige (63) entre la cuvette (53) et la pièce tubulaire (57).

9. Prothèse (65) selon la revendication 1, destinée à une articulation astragalo-calcanéenne, caractérisé en ce que dans chacune des première et seconde parties (68, 69) est formé un puits (10, 20) et le piston (73) comporte un disque (74) de part et d'autre duquel deux tiges déformables (71, 72) font saillie et sont engagées dans les puits correspondants (10, 20) des première et seconde parties, les deux tiges (71 ou 72) pouvant coulisser librement dans les puits correspondants (Fig.10-11).

10. Prothèse (75) selon la revendication 1, destinée à une articulation scapulo-humérale, caractérisée en ce que la première partie est une cupule glénoïdienne (78), pourvue de moyens de fixation à une omoplate (76), tels que des vis (79), ou autres moyens d'ancrage, la seconde partie est une pièce tubulaire (81) prolongée par une pointe (82) destinée à s'ancrer dans l'humérus (77), et cette pièce tubulaire comporte une partie incurvée (83) qui se termine par une tête condylienne (84) présentant une ouverture centrale évasée (85) de passage d'une tige déformable (86) du piston (87), cette tige pouvant librement coulisser à l'intérieur d'un canal (88) de la pièce tubulaire (Fig.12).

11. Prothèse (89) selon la revendication 1, destinée à une articulation radio-carpienne, caractérisée en ce que la première partie comporte une pièce radiale (93) sur laquelle est fixé un plateau glénoïdien (94) et destinée à être ancrée dans un radius (91), des puits (96, 97) de passage d'une tige déformable (98) du piston (99) étant ménagés dans ladite pièce radiale et dans le plateau dans le prolongement l'un de l'autre, la seconde partie est une pièce carpienne (102) dans laquelle est creusé un logement (109) pour une tête (101) du piston (99) fixée dans ce logement, tandis que la tige (98) du piston est librement coulissante dans les puits de la pièce radiale et du plateau (Fig.15-20).

12. Prothèse selon la revendication 11, caractérisée en ce que la pièce carpienne (102) est constituée en deux composants (103, 104) dont une embase (104) équipée d'éléments de fixation dans le carpe, tels que des vis (114), et un secteur sphérique (103) dans lequel est agencé le logement (109) de réception de la tête (101) du piston (99).

13. Prothèse selon l'une des revendciations 11 et 12, caractérisée en ce que le plateau glénoïdien (94) et la pièce carpienne (102) présentent en vis-à-vis des surfaces de frottement complémentaires, à savoir une surface concave (111) sur le plateau et une surface convexe sur la pièce carpienne (102).

14. Prothèse selon l'une quelconque des revendications 1 à 13, caractérisée en ce que les surfaces en frottement mutuel du piston (12, 31...) et de l'une (11, 22, 89,...) desdites parties en matériau biocompatible sont recouvertes d'un matériau à faible coefficient de frottement, par exemple du carbone pyrolytique.

15. Prothèse selon la revendication 2, caractérisée en ce que sa partie trapézienne comprend une coiffe (120) vissée dans une embase (121) et qui assure la retenue de la tête (123) du piston élastique (122), et des moyens de butée (124; 128) sont prévus pour limiter la pénétration de la tige (125) du piston dans la partie métacarpienne (126) (Fig.3 et 4).
